(19) Europäisches Patentamt
European Patent Office
Office européen des brevets

(11) Numéro de publication : 0 364 340 B1

(12) # FASCICULE DE BREVET EUROPEEN

(45) Date de publication du fascicule du brevet : **20.05.92 Bulletin 92/21**

(51) Int. Cl.[5] : **C07C 311/48**

(21) Numéro de dépôt : **89402744.0**

(22) Date de dépôt : **04.10.89**

(54) **Méthode de synthèse de sulfonylimidures.**

(30) Priorité : **05.10.88 FR 8813005**

(43) Date de publication de la demande : **18.04.90 Bulletin 90/16**

(45) Mention de la délivrance du brevet : **20.05.92 Bulletin 92/21**

(84) Etats contractants désignés : **AT BE CH DE ES FR GB IT LI LU NL SE**

(56) Documents cités :
**DE-A- 2 002 065**
**DE-A- 2 002 066**
**DE-A- 2 239 817**

(73) Titulaire : **SOCIETE NATIONALE ELF AQUITAINE**
**Tour Elf, 2, Place de la Coupole, La Défense 6**
**F-92400 Courbevoie (FR)**
Titulaire : **HYDRO-QUEBEC**
**75, Boulevard René Levesque Ouest**
**Montréal Québec H2Z 1A4 (CA)**

(72) Inventeur : **Armand, Michel**
**Les Corjons**
**F-38410 Saint Martin d'Uriage (FR)**

(74) Mandataire : **Boillot, Marc**
**SOCIETE NATIONALE ELF AQUITAINE**
**Division Propriété Industrielle Tour Elf**
**F-92078 Paris la Défense Cédex 45 (FR)**

EP 0 364 340 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention se rapporte à une méthode de synthèse de sulfonylimidures et plus particulièrement de perfluorosulfonylimidures symétriques ou non.

Les perfluorosulfonylimidures répondant à la formule générale $M((R_FSO_2)_2N)_y$, dans laquelle M désigne un métal ou un groupement ammonium quaternaire ou non, les $R_F$, identiques dans le cas d'imidures symétriques ou différents dans le cas d'imidures dissymétriques, représentent des radicaux monovalents perfluorohydrocarbyles et notamment des radicaux perfluoroalcoyles tels que $CF_3$, $C_2F_5$, $C_4F_9$ ou des radicaux perfluoroaryles tels que $CF_5$, et y est un nombre égal à la valence de M, sont intéressants par les propriétés liées à l'anion correspondant. En effet, la délocalisation de la charge de l'anion sur plusieurs centres électronégatifs, à savoir les atomes F, O et N, induit une basicité et un caractère nucléophile faibles. La stabilité des liaisons covalentes permet de plus un domaine étendu de stabilité redox, en particulier aux potentiels anodiques. Les perfluorosulfonylimidures de métaux alcalins et notamment de lithium sont en particulier utilisables pour former des solutions solides avec des matériaux macromoléculaires du type des polyéthers, lesquelles solutions solides trouvent une application comme électrolytes solides polymères dans la production de générateurs primaires ou secondaires tout-solide en films minces (US-A-45-5997).

Les imidures définis par la formule précitée peuvent être obtenus, par exemple, à partir des anhydrides correspondants $(R_FSO_2)_2O$ en faisant appel à l'une ou l'autre des méthodes de synthèse schématisées dans la citation US-A-4505997.

La première de ces méthodes consiste tout d'abord à faire réagir l'anhydride $(R_FSO_2)_2O$ avec l'urée et un acide sulfonique $R_FSO_3H$ selon la réaction:

$$(R_FSO_2)_2\ O+R_FSO_3H+OC(NH_2)_2\text{-->}(R_FSO_2)_2NH+NH_4\ CF_3SO_3+CO_2\uparrow$$

Les produits obtenus après réaction sont ensuite dissous dans l'eau et l'addition de bromure de tétrabutylammonium à la solution obtenue permet de précipiter l'imidure de tétrabutylammonium de formule $(Bu)_4NN(R_FSO_2)_2$. Par une réaction d'échange ionique entre ce composé et le tétraphénylborohydrure de sodium, on forme l'imidure de sodium $Na\ N(R_FSO_2)_2$. Les sels d'autres métaux peuvent se préparer par action des hydroxydes, oxydes ou carbonates des métaux choisis sur l'imidure de sodium.

La seconde desdites méthodes consiste à faire réagir l'anhydride $(R_FSO_2)_2O$ avec la pyridine et le chlorure d'ammonium selon le schéma réactionnel :

$$NH_4CL+2(R_FSO_2)_2O+4\ C_5H_5N\text{--->}C_5H_5NH\ N(R_FSO_2)_2+C_5H_5NHCl+2\ (C_5H_5NH)\ CF_3SO_3$$

Les produits de la réaction sont dissous dans l'eau et l'addition de bromure de tétrabutylammonium à la solution obtenue conduit à la précipitation de l'imidure de tétrabutylammonium. On passe ensuite à l'imidure de sodium puis aux sels d'autres métaux comme indiqué pour la première méthode.

Les méthodes précitées ne sont pas satisfaisantes pour une production des imidures à grande échelle car les rendements globaux sont faibles et les anhydrides précurseurs $(R_FSO_2)_2O$ ne sont pas accessibles aisément.

On peut encore obtenir les imidures mentionnés plus haut à partir des précurseurs $(R_FSO_2F$ en faisant appel à la méthode de synthèse en quatre étapes proposée par J. FOROPOULOS et D.D. DESMARTEAU dans la revue INORGANIC CHEMISTRY, Vol.23 (1984), No.23, pages 3720 à 3723.

Dans cette méthode qui conduit à l'imidure de sodium, le schéma réactionnel est le suivant :

$$1)\quad R_FSO_2F \xrightarrow{NH_3} NH_4NHSO_2R_F \xrightarrow{HCl} H_2NSO_2R_F$$

2) $H_2NSO_2R_F$ + Na $OCH_3$ ------> $NaNHSO_2R_F$ + $HOCH_3$

3) 2 $NaNHSO_2R_F$+$((CH_3)_3Si)_2$ NH-->$(CH_3)_3Si$ $NNaSO_2R_F$+$NH_3\uparrow$

4) $(CH_3)_3Si$ $NNaSO_2R_F$+$R_FSO_2F$-->$NaN(SO_2R_F)_2$ + $(CH_3)_3SiF\uparrow$

Outre un rendement faible, généralement inférieur à 50 %, cette méthode ne peut se généraliser à l'utilisation de précurseurs du type chlorures de sulfonyle $R_FSO_2Cl$ car ces composés ne donnent pas, par action de l'ammoniac, l'amide $H_2NSO_2R_F$ qui est produite dans la première étape de la méthode et qui est nécessaire pour la mise en oeuvre des étapes ultérieures de cette méthode.

Dans la citation DE-A-2239817 on propose une méthode de synthèse de perfluoroalcanesulfonylimidures de métaux alcalins par réaction d'un fluorure de perfluoroalcanesulfonyle sur un dérivé de métal alcalin d'une N-triméthylsilylperfluoroalcanesulfonamide, ledit dérivé de sulfonamide pouvant être obtenu soit par réaction d'un fluorure de perfluoroalcanesulfonyle sur un dérivé de métal alcalin de l'hexaméthyldisilazane ou par réaction d'un dérivé de métal alcalin d'une perfluoroalcanesulfonamide sur l'hexaméthyldisilazane.

Dans la citation DE-A-2002065 on décrit notamment (cf.exemple 18) la préparation de bisméthanesulfonylimide en préparant tout d'abord la N-triméthylsilylméthanesulfonamide par réaction entre l'hexaméthyldisilazane et le chlorure de méthane sulfonyle, puis en faisant réagir ladite sulfonamide avec le chlorure de méthanesulfonyle.

On propose maintenant une méthode de synthèse de sulfonylimidures de formule $M((RSO_2)_2N)y$, dans laquelle les symboles R, identiques ou différents représentent chacun un radical hydrocarbyle et plus particulièrement un radical perfluorohydrocarbyle $R_F$ et M et Y ont les significations données plus haut, à partir de précurseurs du type des fluorures ou des chlorures de sulfonyle correspondants, qui conduit à des rendements élevés, est extrêmement souple de mise en oeuvre à toute échelle et de plus fait appel à des réactifs qui sont d'usage courant et sont disponibles de sources commerciales.

La méthode selon l'invention pour la synthèse de sulfonylimidures de formule $M((RSO_2)_2\ N)_y$, dans laquelle M représente un métal ou un radical ammonium $N(R_1)_4$, les $R_1$ identiques ou différents désignant un atome d'hydrogène ou un radical hydrocarbyle en $C_1$ à $C_8$, les R identiques ou différents représentent chacun un radical organique monovalent en $C_1$ à $C_{12}$ et y est un nombre égal à la valence de M, est du type dans laquelle on fait réagir, d'une part, une composante silazane renfermant un composé silazane de formule $M(((R_2)_3Si)_2\ N)_y$, dans laquelle M et y ont les significations précitées et $R_2$ est un alcoyle en $C_1$ à $C_4$, ou un dérivé silazane de formule $A\ ((R_2)_3Si)_2N$, dans laquelle A représente un atome d'hydrogène ou un radical $(R_2)_3Si$ où $R_2$ a la signification précédente, avec, d'autre part, au moins une composante halogénure de sulfonyle comportant un fluorure de sulfonyle $R\ SO_2F$ ou un chlorure de sulfonyle $R\ SO_2Cl$, R ayant la signification précitée, et elle se caractérise en ce qu'elle consiste à mettre en contact la composante silazane consistant en un composé silazane $M\ [((CR_2)_3Si)_2N]\ y$ avec une composante halogénure de sulfonyle consistant en un fluorure de sulfonyle $RSO_2F$ dans un rapport molaire du composé $RSO_2F$ au composé silazane substantiellement égal à 2y:1 pour former un imidure symétrique en une seule étape ou en ce qu'elle utilise une composante silazane résultant de l'association d'un dérivé silazane $A\ [(R_2)_3Si]_2\ N$ et d'un fluorure $M_1F_z$ ou/et au moins une composante halogénure de sulfonyle résultant de l'association d'un chlorure de sulfonyle $RSO_2Cl$ et d'un fluorure $M_1F_z$, ledit fluorure $M_1F_z$ étant un fluorure de faible énergie réticulaire pour lequel $M_1$ est choisi dans le même groupe que M et peut être identique à M et z et la valence de $M_1$.

Selon un mode de mise en oeuvre, on produit l'imidure en faisant réagir une composante silazane, qui résulte de l'association d'un dérivé silazane $A\ ((R_2)_3Si)_2\ N$ et d'un fluorure $M_1F_z$, avec une composante halogénure de sulfonyle unique consistant en un fluorure de sulfonyle $RSO_2F$ ou avec une première puis une seconde composante halogénure de sulfonyle, dont l'une consiste en un fluorure de sulfonyle $RSO_2F$ et l'autre en un fluorure de sulfonyle $R'SO_2F$, R' appartenant au même groupe que R mais étant différent de ce dernier,le rapport molaire du dérivé silazane au fluorure $M_1F_z$ étant de préférence au plus substantiellement égal à z : 1 lorsque le symbole A désigne un radical $(R_2)_3Si$ ou à 2 z :1 lorsque le symbole A désigne un atome d'hydrogène.

Selon un autre mode de mise en oeuvre, on forme l'imidure en faisant réagir une composante silazane, qui consiste en un composé silazane $M(((R_2)_3Si)_2\ N)y$, sur une composante halogénure de sulfonyle unique résultant de l'association d'un chlorure de sulfonyle $RSO_2Cl$ et d'un fluorure $M_1F_z$ ou sur une première puis une seconde composante halogénure de sulfonyle, dont l'une résulte de l'association d'un chlorure de sulfonyle $RSO_2Cl$ et d'un fluorure $M_1F_z$ tandis que l'autre consiste en un fluorure de sulfonyle $R'SO_2F$ ou résulte de l'association d'un chlorure de sulfonyle $R'SO_2Cl$ et du fluorure $M_1F_z$, R' appartenant au même groupe que R mais étant différent de ce dernier, le rapport molaire du composé $RSO_2Cl$ ou $R'SO_2Cl$ au fluorure $M_1F_z$ étant de préférence au plus substantiellement égal à z :1.

Selon encore un autre mode de mise en oeuvre, on produit l'imidure en faisant réagir une composante silazane, résultant de l'association d'un dérivé silazane $A\ ((R_2)_3\ Si)_2\ N$ et d'un fluorure $M_1F_z$, sur une composante halogénure de sulfonyle unique résultant de l'association d'un chlorure de sulfonyle $RSO_2\ Cl$ et du fluorure $M_1F_z$ ou sur une première puis une seconde composante halogénure de sulfonyle dont l'une résulte de l'association d'un chlorure de sulfonyle $RSO_2Cl$ avec le fluorure $M_1F_z$ et l'autre consiste en un fluorure de sulfonyle $R'SO_2F$ ou résulte de l'association d'un chlorure de sulfonyle $R'SO_2Cl$ et du fluorure $M_1F_z$, R' appartenant au même groupe que R mais étant différent de ce dernier, la quantité molaire c du fluorure $M_1F_z$ à associer aux quantités molaires a du dérivé silazane et b du chlorure ou des chlorures de sulfonyle étant de préférence telle que cest au moins substantiellement égale à $\frac{1}{z}\ x(a+b)$ lorsque le symbole A du dérivé silazane est un radical $(R_2)_3Si$ ou à $\frac{1}{z}\ x\ (\frac{a}{2} + b)$ lorsque le symbole A du dérivé silazane est un atome d'hydrogène.

Les composés silazanes $M(((R_2)_3Si)_2N)_y$ que l'on utilise dans la préparation de l'imidure sont de préférence ceux pour lesquels M représente un métal des groupes I et II de la Classification Périodique des Eléments, notamment Li, Na, K, Ag et Mg, ou un groupement $-NH_4$ ou $-\ N(R_3)_4$, $R_3$ désignant un radical alcoyle en $C_1$ à $C_6$ ou un radical phényle.

Les halogénures de sulfonyle, à savoir fluorures et chlorures de sulfonyle, sont avantageusement choisis parmi ceux pour lesquels R et R′ représentent chacun un radical hydrocarbyle en $C_1$ à $C_{12}$, ledit radical étant plus particulièrement un radical $R_F$ perfluorohydrocarbyle tel qu'un radical perfluoroalcoyle, perfluorocycloalcoyle ou perfluoroaryle. De préférence le radical $R_F$ est un radical perfluoroalcoyle en $C_1$ à $C_8$ tel que $CF_3$, $C_2F_5$, $C_3F_7$, $C_4F_9$, $C_5F_{11}$, $C_6F_3$ ou un radical perfluorophényle $C_6F_5$.

Le radical $R_2$ du composé silazane $M((R_2)_3Si)_2N)_y$ ou du dérivé silazane A $((R_2)_3Si)_2$ N est de préférence un radical méthyle ou éthyle et tout particulièrement un radical méthyle.

Le fluorure $M_1F_z$ à faible énergie réticulaire que l'on emploie dans la méthode de synthèse est par exemple choisi parmi les fluorures KF, AgF et $N(R_4)_4F$, les $R_4$ identiques ou différents, désignant un atome d'hydrogène, un radical alcoyle en $C_1$ à $C_6$ ou un radical phényle.

Selon une forme d'exécution de la méthode selon l'invention, qui conduit à l'obtention d'un imidure symétrique, on fait réagir une composante halogénure de sulfonyle unique avec la composante silazane en quantités telles que le rapport molaire du fluorure ou chlorure de sulfonyle de la composante halogénure de sulfonyle au composé ou dérivé silazane de la composante silazane soit, de préférence, substantiellement égal à :

2 y : 1 lorsque la composante silazane renferme un composé de formule M $((R_2)_3Si)_2$ $N)_y$,

2 : 1 lorsque la composante silazane renferme un dérivé de formule A $((R_2)_3Si)_2N$ pour laquelle A est un radical $(R_2)_3Si$, et

3 : 2 lorsque la composante silazane renferme un dérivé de formule A $((R_2)_3Si)_2N$ pour laquelle A est un atome d'hydrogène.

Selon une autre forme d'exécution de la méthode selon l'invention, qui conduit à l'obtention d'un imidure dissymétrique, on fait réagir, dans une première étape, une première composante halogénure de sulfonyle sur la composante silazane en quantités telles que le rapport molaire du fluorure ou chlorure de sulfonyle de la composante halogénure de sulfonyle au composé ou dérivé silazane soit, de préférence, substantiellement égal à

y : 1 lorsque la composante silazane renferme un composé de formule M $(((R_2)_3Si)_2$ N)y, et

1 : 1 lorsque la composante silazane renferme un dérivé de formule $A((R_2)_3Si)_2$ N, puis, dans une deuxième étape, on met en contact le milieu réactionnel issu de la première étape avec une seconde composante halogénure de sulfonyle, différente de la première et utilisée en quantité molaire substantiellement égale à celle de la première composante halogénure de sulfonyle lorsque la composante silazane de départ renferme un composé M $(((R_2)_3Si)_2N)$y ou un dérivé A $((R_2)_3Si)_2N$ pour lequel A est un radical $(R_2)_3Si$ ou substantiellement égale à la moitié de la quantité de la première composante halogénure de sulfonyle lorsque la composante silazane de départ renferme un dérivé A $((R_2)_3Si)_2N$ pour lequel A est un atome d'hydrogène.

Selon une forme particulière de mise en oeuvre, qui conduit à l'obtention d'un imidure dissymétrique, on fait réagir tout d'abord le chlorure de sulfonyle $RSO_2Cl$ d'une première composante halogénure de sulfonyle, associant ledit chlorure de sulfonyle et un fluorure $M_1F_z$, sur une composante silazane consistant en un composé $M_1(((R_2)_3Si)_2N)_z$, en quantités telles que le rapport molaire du chlorure de sulfonyle au composé silazane soit substantiellement égal à z:1, z étant la valence de $M_1$, puis on met en contact le milieu réactionnel résultant avec une seconde composante halogénure de sulfonyle consistant en un fluorure de sulfonyle $R'\text{–}SO_2F$, R′ désignant un radical choisi dans le même groupe que R mais différent de ce dernier, et avec le fluorure $M_1F_z$ de la première composante halogénure de sulfonyle, en utilisant des quantités des composés $R'SO_2F$ et $M_1F_z$ telles que le rapport molaire $R'SO_2F$ : $RSO_2Cl$ soit substantiellement égal à 1 : 1 et que le rapport molaire $RSO_2Cl$ : $M_1F_z$ soit substantiellement au plus égal à z : 1, l'imidure dissymétrique formé répondant à la formule $M_1(RSO_2\ N\ SO_2R')_z$.

Selon une autre forme particulière de mise en oeuvre, qui conduit également à la production d'un imidure dissymétrique, on fait réagir tout d'abord une première composante halogénure de sulfonyle consistant en un fluorure de sulfonyle $RSO_2F$ avec le dérivé silazane A $((R_2)_3Si)_2N$, qui forme une composante silazane avec un fluorure $M_1F_z$, en quantités telles que le rapport molaire $RSO_2F$ : dérivé silazane soit substantiellement égal à 1:1, puis on ajoute au milieu réactionnel résultant d'une part un chlorure de sulfonyle $R'SO_2Cl$, qui peut également former une composante halogénure de sulfonyle avec le fluorure $M_1F_z$, R′ désignant un radical choisi dans le même groupe que R mais différent de ce dernier, et d'autre part ledit fluorure $M_1F_z$, en utilisant des quantités de chlorure de sulfonyle $R'SO_2Cl$ et de fluorure $M_1F_z$ telles que, lorsque le dérivé silazane a la formule $((R_2)_3Si)_3$ N, le rapport molaire $R'SO_2Cl$ : dérivé silazane soit substantiellement égal à 1:1 et le rapport $R'SO_2Cl$ : $M_1F_z$ soit au plus substantiellement égal à z:2 et que, lorsque le dérivé silazane a la formule $((R_2)_3Si)_2NH$, le rapport molaire $R'SO_2Cl$ : dérivé silazane soit substantiellement égal à 1:2 et le rapport molaire $R'SO_2Cl$ : $M_1F_z$ soit au plus substantiellement égal à z:2, l'imidure produit ayant la formule : $M_1(RSO_2NSO_2R')_z$.

La mise en contact des ingrédients, qui réagissent selon l'invention pour former les imidures, est avantageusement mise en oeuvre dans un milieu solvant constitué d'un solvant aprotique polaire ou d'un mélange de tels solvants. Conviennent en particulier comme solvants aprotiques polaires, des éthers tels que le tétrahydrofuranne, le diglyme et le diméthoxyéthane, des amides tels que le diméthylformamide et la diméthyléthylène urée, des nitriles tels que l'acétonitrile.

Les exemples suivants sont donnés à titre non limitatif pour illustrer l'invention.

EXEMPLE 1 :

A 3,66g de bis(triméthylsilyl)amidure de sodium en solution dans 20 ml de tétrahydrofuranne (THF)anhydre, on ajoutait 6,04 g de fluorure de perfluorobutane sulfonyle. Le mélange ainsi réalisé était agité à température ambiante pendant 48 heures.

La réaction s'effectuait selon le schéma réactionnel suivant :

$$2\ C_4F_9\ SO_2F+Na((CH_3)_3Si)_2N\text{--->}Na(C_4F_9SO_2)_2N+2(CH_3)_3SiF\uparrow$$

L'imidure produit de la réaction, à savoir $Na(C_4F_9SO_2)_2N$, était séparé par évaporation du solvant.

L'acide correspondant à cet imidure, à savoir $H(C_4F_9SO_2)_2N$, peut être extrait, par l'éther éthylique, de la solution aqueuse du sel acidifiée par un acide fort tel que $H_2SO_4$. Les imidures d'autres métaux peuvent alors se préparer par action des hydroxydes, oxydes ou carbonates des métaux appropriés sur l'acide précité.

EXEMPLE 2 :

Dans 50 ml d'une solution 1M du dérivé du potassium de l'hexaméthyldisilazane dans le diméthoxyéthane (DME), maintenue à -20°C, on injectait, sur une période de 3 heures, 15,2 g de fluorure de trifluorométhanesulfonyle. Le milieu réactionnel ainsi formé était ramené à la température ambiante et maintenu à cette température, sous agitation, pendant une période de 12 heures.

L'imidure formé au cours de la réaction, à savoir le bis(trifluorométhanesulfonyl)imidure de potassium de formule $K(CF_3SO_2)_2N$, était isolé avec un rendement de 83 % après évaporation du solvant.

La réaction s'effectuait selon le schéma réactionnel suivant :

$$2\ CF_3SO_2F + K((CH_3)_3Si)_2N\ \text{---->}\ (CF_3SO_2)_2N+2(CH_3)_3SiF\uparrow.$$

Le sel de potassium de l'hexaméthyldisilazane était préparé par réaction de l'hexaméthyldisilazane sur l'hydrure de potassium.

L'acide $H(CF_3SO_2)_2N$ peut être obtenu pur par traitement de l'imidure $K(CF_3SO_2)_2N$ par l'acide sulfurique anhydre et distillation sous pression réduite (0,3 Pa à 90°C), ledit acide se présentant comme un solide hygroscopique fondant à 30-35°C. Les imidures de métaux divers dérivant dudit acide peuvent être obtenus par réaction de cet acide avec les hydroxydes, oxydes ou carbonates des métaux appropriés.

EXEMPLE 3 :

A 50 ml d'une solution 1 M du dérivé du potassium de l'hexaméthyldisilazane dans le DME, maintenue à -20°C, on ajoutait 30,2 g de fluorure de perfluorobutanesulfonyle en solution dans 20 ml de DME, ladite addition étant réalisée sur une période de 3 heures. Le milieu réactionnel obtenu était ensuite ramené à la température ambiante et maintenu à cette température, sous agitation, pendant 12 heures.

L'imidure formé au cours de la réaction, à savoir le bis(perfluorobutanesulfonyl)imidure de potassium de formule $K(C_4F_9SO_2)_2N$, était isolé par distillation du solvant DME, puis purifié par recristallisation dans l'eau.

La réaction s'effectuait selon le schéma réactionnel ci-après :

$$2\ C_4F_9SO_2F+K((CH_3)_3Si)_2N\ \text{--->}\ K(C_4F_9SO_2)_2\ N+2(CH_3)_3SiF\uparrow$$

EXEMPLE 4 :

Dans 100 ml d'une solution 1 M du dérivé lithié de l'hexaméthyldisilazane dans le THF, maintenue à -18°C, on injectait lentement 30,4 g de fluorure de trifluorométhanesulfonyle. Après retour du milieu réactionnel ainsi obtenu à la température ambiante, on évaporait le solvant dudit milieu pour laisser un résidu solide de bis(trifluorométhanesulfonyl)-imidure de lithium. Après purification par lavage au dichlorométhane, on obtenait 26 g dudit composé lithié.

La réaction s'effectuait selon le schéma réactionnel suivant :

$$2\ CF_3SO_2F+Li\ ((CH_3)_3Si)_2N\ \text{--->}\ Li(CF_3SO_2)_2N+2\ (CH_3)_3SiF\uparrow$$

EXEMPLE 5

On dissolvait 2,9 g du composé $Li(CF_3SO_2)_2N$, obtenu comme décrit à l'exemple 4, avec 4,4 g d'un polymère d'oxyde d'éthylène ayant une masse moléculaire égale à $5x10^5$ dans 200 ml d'acétonitrile. Une fraction

égale à 15 ml de la solution visqueuse obtenue était coulée sur une plaque de polytétrafluoroéthylène dans une anneau de verre d'un diamètre égal à 50 mm.

Après évaporation du solvant dans une étuve à 60°C, on obtenait un film élastique et amorphe de 220 μm d'épaisseur du complexe Li $(CF_3SO_2)_2$ N/polymère. Ce matériau présente à 25°C une conductivité ionique égale à 2 x $10^5$ $Ohm^{-1}cm^{-1}$ et peut être utilisé pour la constitution de la couche d'électrolyte solide et également pour la réalisation du liant de l'électrode positive composite de générateurs en films minces tout-solide primaires ou secondaires dont l'électrode négative est constituée de lithium métallique ou d'un alliage à base de lithium tel qu'un alliage lithium-aluminium.

EXEMPLE 6 :

A 5,41 ml de nonaméthyltrisilazane dans 15 ml de DME, on ajoutait 1,16 g de fluorure de potassium KF anhydre. Le mélange ainsi formé était refroidi à -20°C. Au mélange refroidi on ajoutait alors 6,08 g de fluorure de trifluorométhanesulfonyle. Après retour du milieu réactionnel à température ambiante et distillation du solvant, on obtenait 3 g de bis(trifluorométhanesulfonyl) imidure de potassium.

La réaction s'effectuait selon le schéma réactionnel suivant :

$$2\ CF_3SO_2F + ((CH_3)_3Si)_3\ N + KF \text{---}> K(CF_3SO_2)_2\ N + 3\ (CH_3)_3SiF\uparrow$$

EXEMPLE 7 :

A 25 ml d'une solution 0,5 M de fluorure de tétrabutylammonium dans le THF, on ajoutait 3,38 ml de nonaméthyltrisilazane, puis 5,05 g de fluorure de pentafluoroéthanesulfonyle. Le milieu réactionnel était maintenu sous agitation à température ambiante pendant environ 1,5 heures, puis le solvant était évaporé. Après évaporation du solvant on obtenait alors 7 g de bis(pentafluoroéthanesulfonyl)-imidure de tétrabutylammonium de formule $(C_4H_9)_4\ N(C_2F_5SO_2)_2N$.

La réaction s'effectuait selon le schéma réactionnel suivant :

$$2\ C_2F_5SO_2F + ((CH_3)_3Si)_3\ N + (C_4H_9)_4\ NF \text{-------}> (C_4H_9)_4\ N(C_2F_5SO_2)_2\ N + 3\ (CH_3)_3SiF\uparrow$$

EXEMPLE 8 :

A 10 ml d'une solution 1M du dérivé lithié de l'hexaméthyldisilazane dans le DME, on ajoutait 1,74 g de KF anhydre, puis 2,2 ml de chlorure de trifluorométhane sulfonyle. Le milieu réactionnel était ensuite maintenu sous agitation à température ambiante pendant 2 heures.

La réaction s'effectuait selon le schéma réactionnel suivant :

$$2\ CF_3SO_2Cl + 3KF + Li((CH_3)_3Si)_2N \text{--------}> K(CF_3SO_2)_2\ N + 2KCl + LiF + 2(CH_3)_3SiF\uparrow$$

A l'issue de la réaction, les sels KCl et LiF étaient séparés du milieu réactionnel par filtration et la solution obtenue était alors soumise à évaporation pour éliminer le solvant et isoler l'imidure comme résidu de l'évaporation.

On obtenait à l'issue de l'évaporation 3,1 g d'imidure de formule K $(CF_3\ SO_2)_2$ N.

EXEMPLE 9 :

A une solution de 9,55 g de chlorure de perfluorobutanesulfonyle dans 30 ml de DME, on ajoutait 4,25 ml d'hexaméthyldisilazane et 1,48 g de $NH_4F$. Le milieu réactionnel ainsi formé était maintenu sous agitation à température ambiante pendant 6 heures.

La réaction s'effectuait selon le schéma réactionnel suivant :

$$3\ C_4F_9SO_2Cl + 4\ NH_4F + 2\ ((CH_3)_3Si)_2NH \text{-----}> C_4F_9SO_2NH_2 + 3NH_4Cl + NH_4(C_4F_9SO_2)_2\ N + 4(CH_3)_3SiF\uparrow$$

Le chlorure d'ammonium se présentait sous la forme d'un précité que l'on séparait du milieu réactionnel par filtration.

La solution résiduelle était soumise à une évaporation pour éliminer le solvant et former un résidu d'évaporation consistant en un mélange de l'imidure $NH_4\ (C_4F_9SO_2)_2N$ et de l'amide $C_4F_9SO_2NH_2$. La reprise du résidu sec de l'évaporation par le dichlorométhane, suivie d'une filtration permet de séparer l'imidure qui est insoluble dans ce solvant.

EXEMPLE 10 :

A une solution de 22 ml de chlorure de trifluorométhanesulfonyle dans 200 ml de DME on ajoutait 17,5 g de KF anhydre, puis 18,9 ml de nonaméthyltrisilazane. Le mélange était maintenu sous agitation à température

ambiante pendant 72 heures.

La réaction s'effectuait selon le schéma réactionnel suivant :

$$2CF_3SO_2Cl+3KF+((CH_3)_3Si)_3N\text{->}2KCl+K(CF_3SO_2)_2N+3(CH_3)_3SiF\uparrow$$

A l'issue de la période de réaction, le milieu réactionnel était filtré pour séparer KCl insoluble dans ledit milieu. Par évaporation du filtrat, on obtenait 31 g du sel de potassium du bis(trifluorométhanesulfonyl)imidure.

EXEMPLE 11 :

A une solution de 4,4 ml de chlorure de trifluorométhane sulfonyle dans 25 ml d'acétonitrile, on ajoutait 5,4 ml de nonaméthyltrisilazane, puis 7,56 g de fluorure d'argent anhydre. Le milieu réactionnel ainsi formé était alors maintenu sous agitation à température ambiante pendant 2 heures.

La réaction s'effectuait selon le schéma réactionnel suivant :

$$2\ CF_3SO_2Cl + ((CH_3)_3Si)_3\ N + 3\ AgF\text{--->}Ag(CF_3SO_2)_2N+2AgCl+3(CH_3)_3SiF\uparrow$$

A l'issue de la période de réaction, le milieu réactionnel était filtré pour séparer AgCl insoluble dans ledit milieu. Par évaporation du filtrat pour chasser le solvant, on obtenait l'imidure $Ag(CF_3SO_2)_2$ N comme résidu d'évaporation. Cet imidure est très soluble dans les solvants aromatiques tels que benzène et toluène.

EXEMPLE 12 :

A 10 ml d'une solution 1M d'hexaméthyldisilylamidure de potassium dans le diglyme, on ajoutait 2,67 g de chlorure de pentafluorobenzène sulfonyle. Un précité de KCl se formait.

La réaction s'effectuait selon le schéma réactionnel ci-après :

$$C_6F_5SO_2Cl+K((CH_3)_3Si)_2N\text{---->}KCl + C_6F_5SO_2N(Si(CH_3)_3)_2$$

Au milieu réactionnel ainsi formé, on ajoutait 0,58 g de KF anhydre et 3,02 g de fluorure de perfluorobutanesulfonyle et maintenait le tout sous agitation à température ambiante pendant 3 heures.

Il se formait un imidure mixte selon le schéma réactionnel suivant :

$$C_6F_5SO_2N(Si(CH_3)_3)_2 + KF + C_4F_9\ SO_2F\text{ ---->} K\ (C_6F_5\ SO_2\ N\ SO_2C_4F_9) + 2(CH_3)_3SiF\uparrow$$

A l'issue de la période de réaction, le milieu réactionnel était filtré pour séparer le KCl insoluble dans ledit milieu. Par distillation du filtrat sous pression réduite pour chasser le solvant, on isolait l'imidure mixte précité comme résidu de la distillation.

EXEMPLE 13 :

A 20 ml de THF maintenus à - 18°C, on ajoutait 4,4 ml de nonaméthylsilazane, puis 3,04 g de fluorure de trifluorométhanesulfonyle. Après retour à la température ambiante et agitation pendant 2 heures, on ajoutait alors au milieu réactionnel 2,32 g de KF anhydre et 4,37 g de chlorure de pentafluoroéthanesulfonyle et maintenait le tout sous agitation à température ambiante pendant 2 heures.

Il se formait un imidure mixte selon le schéma réactionnel suivant :

1) $CF_3SO_2F+((CH_3)_3Si)_3N\text{--->}CF_3SO_2N(Si(CH_3)_3)_2+(CH_3)_3SiF\uparrow$

2) $CF_3\ SO_2\ N\ (Si(CH_3)_3)_2 + 2\ KF + C_2F_5\ SO_2\ Cl\text{ ---->} KCl + 2\ (CH_3)_3SiF\uparrow + K(CF_3SO_2\ N\ SO_2\ C_2F_5)$

A l'issue de la période de réaction, le milieu réactionnel était filtré pour séparer KCl insoluble dans ledit milieu. Le filtrat était alors évaporé pour chasser le solvant et l'on obtenait l'imidure mixte ci-dessus comme résidu de l'évaporation.

## Revendications

1. Méthode de synthèse de sulfonylimidures de formule générale $M((RSO_2)_2N)_y$, où M représente un métal ou un radical ammonium $N(R_1)_4$, les $R_1$ identiques ou différents désignant un atome d'hydrogène ou un radical hydrocarbyle en $C_1$ à $C_8$, les R, identiques ou différents, représentent chacun un radical organique monovalent en $C_1$ à $C_{12}$ et y est un nombre égal à la valence de M, dans laquelle on fait réagir, d'une part, une composante silazane renfermant un composé silazane de formule $M(((R_2)_3Si)_2N)_y$, dans laquelle M et Y ont les significations précitées et $R_2$ est un alcoyle en $C_1$ à $C_4$, ou un dérivé silazane de formule $A((R_2)_3Si)_2N$, dans laquelle A représente un atome d'hydrogène ou un radical $(R_2)_3Si$ où $R_2$ a la signification précédente, avec, d'autre part, au moins une composante halogénure de sulfonyle comportant fluorure de sulfonyle $RSO_2F$ ou un chlorure de sulfonyle $RSO_2Cl$, R ayant la signification précitée, ladite méthode se caractérisant en ce qu'elle consiste à mettre en contact la composante silazane consistant en un composé silazane $M\ [((R_2)_3Si)_2N]_y$ avec une composante halogénure de sulfonyle consistant en un fluorure de sulfonyle $RSO_2F$ dans un rapport molaire du

composé $RSO_2F$ au composé silazane substantiellement égal à 2y:1 pour former un imidure symétrique ou en ce qu'elle utilise une composante silazane résultant de l'association d'un dérivé silazane A $((R_2)_3Si)_2N$ et d'un fluorure $M_1F_z$ ou/et au moins une composante halogénure de sulfonyle résultant de l'association d'un chlorure de sulfonyle $RSO_2Cl$ et d'un fluorure $M_1F_z$, ledit fluorure $M_1F_z$ étant un fluorure de faible énergie réticulaire pour lequel $M_1$ est choisi dans le même groupe que M et peut être identique à ce dernier et z est la valence de $M_1$.

2. Méthode selon la revendication 1, caractérisée en ce que l'on produit l'imidure en faisant réagir une composante silazane, qui résulte de l'association d'un dérivé silazane A $((R_2)_3Si)_2N$ et d'un fluorure $M_1F_z$, avec une composante halogénure de sulfonyle unique consistant en un fluorure de sulfonyle $RSO_2F$ ou avec une première puis une seconde composante halogénure de sulfonyle, dont l'une consiste en un fluorure de sulfonyle $RSO_2F$ et l'autre en un fluorure de sulfonyle $R'SO_2F$, R' appartenant au même groupe que R mais étant différent de ce dernier, le rapport molaire du dérivé silazane au fluorure $M_1F_z$ étant au plus substantiellement égal à z:1 lorsque le symbole A désigne un radical $(R_2)_3Si$ ou à 2z:1 lorsque le symbole A désigne un atome d'hydrogène.

3. Méthode selon la revendication 1, caractérisée en ce que l'on produit l'imidure en faisant réagir une composante silazane, qui consiste en un composé silazane $M(((R_2)_3Si)_2N)_y$, sur une composante halogénure de sulfonyle unique, qui résulte de l'association d'un chlorure de sulfonyle $RSO_2Cl$ et d'un fluorure $M_1F_z$ ou sur une première puis une seconde composante halogénure de sulfonyle, dont l'une résulte de l'association d'un chlorure de sulfonyle $RSO_2Cl$ et d'un fluorure $M_1F_z$ tandis que l'autre consiste en un fluorure de sulfonyle $R'-SO_2F$ ou résulte de l'association d'un chlorure de sulfonyle $R'SO_2Cl$ et du fluorure $M_1F_z$, R' appartenant au même groupe que R mais étant différent de ce dernier, le rapport molaire du composé $RSO_2Cl$ ou $R'SO_2Cl$ au fluorure $M_1F_z$ étant au plus substantiellement égal à z : 1.

4. Méthode selon la revendication 1, caractérisée en ce que l'on produit l'imidure en faisant réagir une composante silazane, résultant de l'association d'un dérivé silazane $A((R_2)_3Si)_2N$ et d'un fluorure $M_1F_z$, sur une composante halogénure de sulfonyle unique résultant de l'association d'un chlorure de sulfonyle $RSO_2Cl$ et du fluorure $M_1F_z$ ou sur une première puis une seconde composante halogénure de sulfonyle, dont l'une résulte de l'association d'un chlorure de sulfonyle $RSO_2Cl$ avec le fluorure $M_1F_z$ et l'ature consiste en un fluorure de sulfonyle $R'SO_2F$ ou résulte l'association d'un chlorure de sulfonyle $R'SO_2Cl$ et du fluorure $M_1F_z$, R' appartenant au même groupe que R mais étant différent de ce dernier, la quantité molaire c du fluorure $M_1F_z$ à associer aux quantités molaires a du dérivé silazane et b du chlorure ou des chlorures de sulfonyle étant telle que cest au moins substantiellement égale à $\frac{1}{z}x(a+b)$ lorsque le symbole A du dérivé silazane est un radical $(R_2)_3Si$ et $\frac{1}{z}x(\frac{a}{2}+b)$ lorsque le symbole A du dérivé silazane est un atome d'hydrogène.

5. Méthode selon la revendication 1 ou 3, caractérisée en ce que le composé silazane $M(((R_2)_3Si)_2\,N)_y$ est choisi parmi ceux pour lesquels M représente un métal des groupes I et II de la Classification Périodique des Eléments, notamment Li, Na, K, Ag et Mg, ou un groupement $NH_4$ ou - $N(R_3)_4$, $R_3$ désignant un radical alcoyle en $C_1$ à $C_6$ ou un radical phényle.

6. Méthode selon l'une des revendications 1 à 5, caractérisée en ce que les fluorures et chlorures de sulfonyle sont choisis parmi ceux pour lesquels R et R' représentent chacun un radical hydrocarbyle en $C_1$ à $C_{12}$ et de préférence un radical perfluorohydrocarbyle $R_F$ en $C_1$ à $C_{12}$ tel qu'un radical perfluoroalcoyle, perfluorocycloalcoyle ou perfluoroaryle.

7. Méthode selon la revendication 6, caractérisée en ce que le radical $R_F$ est un radical perfluoroalcoyle en $C_1$ à $C_8$ ou un radical perfluorophényle.

8. Méthode selon l'une des revendications 1 à 7, caractérisée en ce que le radical $R_2$ du composé silazane est un radical méthyle ou éthyle et de préférence un radical méthyle.

9. Méthode selon l'une des revendications 1 à 8, caractérisée en ce que le fluorure $M_1F_z$ associé au dérivé silazane et au chlorure de sulfonyle est choisi parmi les fluorures KF, AgF et $N(R_4)_4F$, les $R_4$ identiques ou différents désignant un atome d'hydrogène, un radical alcoyle en $C_1$ à $C_6$ ou un radical phényle.

10. Méthode selon l'une des revendications 2 à 9, caractérisée en ce que, pour former un imidure symétrique, on fait réagir une composante halogénure de sulfonyle unique avec la composante silazane en quantités telles que le rapport molaire du fluorure de sulfonyle $RSO_2F$ ou du chlorure de sulfonyle $RSO_2Cl$ de la composante halogénure de sulfonyle au composé ou dérivé silazane soit substantiellement égal :

à 2y:1 lorsque la composante silazane renferme un composé de formule $M((R_2)_3Si)_2\,N)_y$,

à 2:1 lorsque la composante silazane renferme un dérivé de formule $A((R_2)_3Si)_2N$ pour laquelle A est un radical $(R_2)_3Si$, et

à 3:2 lorsque la composante silazane renferme un dérivé de formule $A((R_2)_3Si)_2N$ pour laquelle A est un atome d'hydrogène.

11. Méthode selon l'une des revendications 2 à 9, caractérisée en ce que, pour former un imidure dissymétrique, on fait réagir, dans une première étape, une première composante halogénure de sulfonyle sur la

composante silazane en quantités telles que le rapport molaire du fluorure ou chlorure de sulfonyle de la composante halogénure de sulfonyle au composé ou dérivé silazane soit substantiellement égal :
à y:1 lorsque la composante silazane renferme un composé de formule $M(((R_2)_3Si)_2N)_y$, et
à 1:1 lorsque la composante silazane renferme un dérivé de formule $A((R_2)_3Si)_2N$,
puis, dans une deuxième étape, on met en contact le milieu réactionnel issu de la première étape avec une seconde composante halogénure de sulfonyle, différente de la première et utilisée en quantité molaire substantiellement égale à celle de la première composante halogénure de sulfonyle lorsque la composante silazane de départ renferme un composé $M(((R_2)_3Si)_2N)_y$ ou un dérivé $A((R_2)_3Si)_2N$ pour lequel A est un radical $(R_2)_3Si$ ou substantiellement égale à la moitié de la quantité de la première composante halogénure de sulfonyle lorsque la composante silazane de départ renferme un dérivé $A((R_2)_3Si)_2N$ pour lequel A est un atome d'hydrogène.

12. Méthode selon la revendication 1, caractérisée en ce que, pour former un imidure dissymétrique, on fait réagir tout d'abord le chlorure de sulfonyle $RSO_2Cl$ d'une première composante halogénure de sulfonyle, associant ledit chlorure de sulfonyle et un fluorure $M_1F_z$, sur une composante silazane consistant en un composé $M_1(((R_2)_3Si)_2N)_z$, en quantités telles que le rapport molaire du chlorure de sulfonyle au composé silazane soit substantiellement égal à z:1, z étant la valence de $M_1$, puis on met en contact le milieu réactionnel résultant avec une seconde composante halogénure de sulfonyle consistant en un fluorure de sulfonyle R'–$SO_2F$, R' étant un radical choisi dans le même groupe que R mais différent de ce dernier, et avec le fluorure $M_1F_z$ de la première composante halogénure de sulfonyle, en utilisant des quantités des composés $R'SO_2F$ et $M_1F_z$ telles que le rapport molaire $R'SO_2F$ : $RSO_2Cl$ soit substantiellement égal à 1:1 et que le rapport molaire $RSO_2Cl$ : $M_1F_z$ soit substantiellement au plus égal à z:1, l'imidure dissymétrique formé répondant à la formule $M_1$ $(RSO_2NSO_2R')_z$.

13. Méthode selon la revendication 1, caractérisée en ce que, pour former un imidure dissymétrique, on fait réagir tout d'abord une première composante halogénure de sulfonyle consistant en un fluorure de sulfonyle $RSO_2F$ avec un dérivé silazane $A((R_2)_3Si)_2N$, qui forme une composante silazane avec un fluorure $M_1F_z$, en quantités telles que le rapport molaire R $SO_2F$ : dérivé silazane soit substantiellement égal à 1:1, puis on ajoute au milieu réactionnel résultant, d'une part, un chlorure de sulfonyle $R'SO_2Cl$, qui peut également former une composante halogénure de sulfonyle avec le fluorure $M_1F_z$, R' étant un radical choisi dans le même groupe que le radical R mais différent de ce dernier, et, d'autre part, ledit fluorure $M_1F_z$, en utilisant des quantités de chlorure de sulfonyle $R'SO_2Cl$ et de fluorure $M_1F_z$ telles que, lorsque le dérivé silazane a la formule $((R_3)_3Si)_3N$, le rapport molaire $R'SO_2Cl$: dérivé silazane soit substantiellement égal à 1:1 et le rapport molaire $R'SO_2Cl$ : $M_1F_z$ soit au plus substantiellement égal à z:2 et que, lorsque le dérivé silazane a la formule $((R_2)_3Si)_2)NH$, le rapport molaire $R'SO_2Cl$ : dérivé silazane soit substantiellement égal à 1:2 et le rapport molaire $R'SO_2Cl$ : $M_1F_z$ soit au plus substantiellement égal à z:2, l'imidure produit ayant la formule $M_1$ $(RSO_2\ N\ SO_2R')_z$.

14. Méthode selon l'une des revendications 1 à 13, caractérisée en ce que la mise en contact des ingrédients, qui réagissent pour former les imidures, est mise en oeuvre dans un milieu solvant constitué d'un solvant aprotique polaire ou d'un mélange de tels solvants.

## Patentansprüche

1. Verfahren zur Herstellung von Sulfonylimiden der allgemeinen Formel $M((RSO_2)_2N)_y$,worin M ein Metall oder einen Ammoniumrest $N(R_1)_4$ darstellt, die Reste $R_1$ gleich oder verschieden sind und ein Wasserstoffatom oder einen $C_1$-$C_8$-Kohlenwasserstoffrest bedeuten, die Reste R gleich oder verschieden sind und jeweils einen einwertigen organischen $C_1$-$C_{12}$-Rest bedeuten und y eine der Wertigkeit von M entsprechende Zahl bedeutet, durch Umsetzung einer Silazankomponente, die eine Silazanverbindung der Formel $M(((R_2)_3Si)_2N)_y$, worin M und y die angeführten Bedeutungen haben und $R_2$ ein $C_1$-$C_4$-Alkyl darstellt, oder ein Silazan derivat der Formel $A((R_2)_3Si)_2N$ enthält, worin A ein Wasserstoffatom oder einen Rest $(R_2)_3Si$ darstellt, worin $R_2$ die angeführte Bedeutung hat, mit wenigstens einer Sulfonylhalogenidkomponente, die Sulfonylfluorid $RSO_2F$ oder ein Sulfonylchlorid $RSO_2Cl$ enthält, wobei R die angeführte Bedeutung hat, dadurch **gekennzeichnet**, daß man die in einer Silazanverbindung $M[((R_2)_3Si)_2N]_y$ bestehende Silazankomponente mit einer in einem Sulfonylfluorid $RSO_2F$ bestehenden Sulfonylhalogenidkomponente bei einem Molarverhältnis von $RSO_2F$ zur Silazanverbindung im wesentlichen von 2y:1 umsetzt, um ein symmetrisches Imid zu bilden oder man eine Silazankomponente verwendet, die das Ergebnis der Assoziation eines Silazanderivats A $((R_2)_3Si)_2N$ mit einem Fluorid $M_1F_2$ und/oder wenigstens eine Sulfonylhalogenidverbindung, die das Ergebnis der Assoziation eines Sulfonylchlorids $RSO_2Cl$ mit einem Fluorid $M_1F_2$ ist, wobei das Fluorid $M_1F_2$ ein Fluorid von schwacher Gitterenergie ist, wobei $M_1$ ausgewählt ist unter derselben Gruppe wie M und mit diesem identisch sein kann und z die Wertigkeit von $M_1$ bedeutet.

2. Verfahren nach Anspruch 1, dadurch **gekennzeichnet**, daß man das Imid herstellt durch Umsetzung

einer Silazankomponente, die man durch Assoziation eines Silazanderivats $A((R_2)_3Si)_2N$ mit einem Fluorid $M_1F_2$ erhält, mit einer einzigen Sulfonylhalogenidkomponente, die in einem Sulfonylfluorid $RSO_2F$ besteht, oder mit einer ersten Sulfonylhalogenidkomponente und dann mit einer zweiten, von denen die eine ein Sulfonylfluorid $RSO_2F$ darstellt und die andere ein Sulfonylfluorid $R'SO_2F$, wobei R′ zur selben Gruppe wie R gehört, sich von diesem jedoch unterscheidet, und das Molarverhältnis des Silazanderivats zum Fluorid $M_1F_2$ im wesentlichen höchstens höchstens z:1 ist, wenn a einen Rest $(R_2)_3Si$ bedeutet oder 2z:1, wenn A ein Wasserstoffatom bedeutet.

3. Verfahren nach Anspruch 1, dadurch **gekennzeichnet**, daß man das Imid herstellt durch Umsetzung einer Silazankomponente, die in einer Silazanverbindung $M[((R_2)_3Si)_2N]_y$ besteht, mit einer einzigen Sulfonylhalogenidkomponente, die man durch Assoziation eines Sulfonylchlorids $RSO_2Cl$ mit einem Fluorid $M_1F_2$ erhält, oder mit einer ersten Sulfonylhalogenidkomponente und dann mit einer zweiten, deren eine man durch Assoziation eines Sulfonylchlorids $RSO_2Cl$ mit einem Fluorid $M_1F_2$ erhält, während die andere in einem Sulfonylfluorid $R'SO_2F$ besteht, oder durch Assoziation eines Sulfonylchlorids $R'SO_2Cl$ mit einem Fluorid $M_1F_2$, wobei R′ zur selben Gruppe wie R gehört, sich von diesem jedoch unterscheidet, und das Molarverhältnis der Verbindung $RSO_2Cl$ oder $R'SO_2Cl$ zum Fluorid $M_1F_2$ im wesentlichen höchstens z:1 ist.

4. Verfahren nach Anspruch 1, dadurch **gekennzeichnet**, daß man das Imid herstellt durch Umsetzung einer Silazankomponente, die man durch Assoziation eines Silazanderivats A $((R_2)_3Si)_2N$ mit einem Fluorid $M_1F_z$ erhält, mit einer einzigen Sulfonylhalogenidkomponente, die man durch Assoziation eines Sulfonylchlorids $RSO_2Cl$ mit eineml Fluorid $M_1F_z$ erhält oder mit einer ersten Sulfonylhalogenidkomponente und dann mit einer zweiten, deren eine man durch Assoziation eines Sulfonylchlorids $RSO_2Cl$ mit einem Fluorid $M_1F_z$ erhält und deren andere in einem Sulfonylfluorid $R'SO_2F$ besteht, oder sie durch Assoziation eines Sulfonylchlorids $RSO_2Cl$ mit einem Fluorid $M_1F_z$, wobei R′ zur selben Gruppe wie R gehört, sich von diesem jedoch unterscheidet, und die mit den Molarmengen a des Silazanderivats und b des Sulfonylchlorids bzw. der Sulfonylchloride zu assoziierende Molarmenge c des Fluorids $M_1F_z$ so gewählt ist, daß sie wenigstens im wesentlichen gleich 1/z x (a + b) ist, wenn A des Silazanderivats ein Rest $(R_2)_3Si$ ist und gleich 1/z x (a/2 + b) ist, wenn A des Silazanderivats ein Wasserstoffatom ist.

5. Verfahren nach Anspruch 1 oder 3, dadurch **gekennzeichnet**, daß die Silazanverbindung $M(((R_2)_3Si)_2N)_y$ unter Silazanverbindungen ausgewählt ist, bei denen M ein Metall der I. und II. Gruppe des Periodensystems darstellt, und zwar Li, Na, K, Ag und Mg, oder eine Gruppe $NH_4$ oder - $N(R_3)_4$, wobei $R_3$ einen $C_1$-$C_6$-Alkylrest oder einen Phenylrest bedeutet.

6. Verfahren nach einem der Ansprüche 1 bis 5, dadurch **gekennzeichnet**, daß die Sulfonylfluoride und -chloride ausgewählt sind unter solchen, bei denen R und R′ jeweils einen $C_1$-$C_{12}$-Kohlenwasserstoffrest und vorzugsweise einen $C_1$-$C_{12}$-Perfluorkohlenwasserstoffrest R, wie einen Perfluoralkyl-, Perfluorcycloalkyl- oder Perfluorarylrest darstellen.

7. Verfahren nach Anspruch 6, dadurch **gekennzeichnet**, daß der Rest R, ein $C_1$-$C_8$-Perfluoralkyl- oder Perfluorphenylrest ist.

8. Verfahren nach einem der Ansprüche 1 bis 7, dadurch **gekennzeichnet** , daß der Rest $R_2$ der Silazanverbindung ein Methyl- oder Ethylrest und vorzugsweise ein Methylrest ist.

9. Verfahren nach einem der Ansprüche 1 bis 8, dadurch **gekennzeichnet**, daß das mit dem Silazanderivat und dem Sulfonylchlorid assoziierte Fluorid $M_1F_z$ ausgewählt wird unter den Fluoriden KF, AgF und $N(R_4)_4F$, die Reste $R_4$ gleich oder verschieden sind und ein Wasserstoffatom, ein $C_1$-$C_6$-Alkyl oder einen Phenylrest bedeuten.

10. Verfahren nach einem der Ansprüche 2 bis 9, dadurch **gekennzeichnet** , daß man zur Bildung eines symmetrischen Imids eine einzige Sulfonylhalogenidkomponente mit der Silazankomponente in solchen Mengen umsetzt, daß das Molarverhältnis von Sulfonylfluorid $RSO_2F$ oder von Sulfonylchlorid $RSO_2Cl$ der Sulfonylhalogenidkomponente zur Silazanverbindung bzw. seinem Derivat im wesentlichen gleich
2y:1 beträgt, wenn die Silazankomponente eine Verbindung der Formel $M(((R_2)_3Si)_2N)_y$ enthält,
2:1 wenn die Silazankompomente ein Derivat der Formel $A((R_2)_3Si)_2N$ enthält, wobei A ein Rest $(R_2)_3Si$ ist, und
3:2, wenn die Silazankomponente ein Derivat der Formel $A((R_2)_3Si)_2N$ enthält, wobei A eine Wasserstoffatom ist.

11. Verfahren nach einem der Ansprüche 2 bis 9, dadurch **gekennzeichnet** , daß man zur Bildung eines asymmetrischen Imids in einer ersten Stufe eine erste Sulfonylhalogenidkomponente mit der Silazankomponente in solchen Mengen umsetzt, daß das Molarverhältnis von Sulfonylfluorid oder -chlorid der Sulfonylhalogenidkomponente zur Silazanverbindung bzw. ihrem Derivat im wesentlichen gleich
y:1 beträgt, wenn die Silazankomponente eine Verbindung der Formel $M(((R_2)_3Si)_2N)_y$ enhält,
1:1 wenn die Silazankomponente ein Derivat der Formel $A((R_2)_3Si)_2N$ enthält, und dann in einer zweiten Stufe das Reaktionsmedium aus der ersten Stufe mit einer zweiten Sulfonylhalogenidkomponente kontaktiert, die sich von der ersten unterscheidet und in einer mit der ersten Sulfonylhalogenidkomponente im wesentlichen

gleichen molaren Menge verwendet wird, wenn die Ausgangssilazankomponente eine Verbindung $M(((R_2)_3Si)_2N)_y$ oder ein Derivat $A((R_2)_3Si)_2N$ ist, wobei A ein Rest $(R_2)_3Si$ ist, oder in einer im wesentlichen der halben Menge der ersten Sulfonylhalogenidkomponente entsprechenden Menge, wenn die Ausgangssilazankomponente ein Derivat der Formel $A((R_2)_3Si)_2N$ enthält, wobei A ein Wasserstoffatom ist.

12. Verfahren nach Anspruch 1, dadurch **gekennzeichnet**, daß man zur Bildung eines asymmetrischen Imids zuerst das Sulfonylchlorid $RSO_2Cl$ einer ersten durch Assoziierung des Sulfonylchlorids mit einem Fluorid $M_1F_z$ zu bildenden Sulfonylhalogenidkomponente mit einer Silazankomponente, die in einer Verbindung der Formel $M_1(((R_2)_3Si)_2N)_2$ besteht, in solchen Mengen umsetzt, daß das Molarverhältnis des Sulfonylchlorids zur Silazanverbindung im wesentlichen gleich z:1 ist, wobei z die Wertigkeit von $M_1$ ist, und dann das erhaltene Reaktionsmedium mit einer zweiten Sulfonylhalogenidkomponente, die in einem Sulfonylfluorid $R'SO_2F$ besteht, wobei R′ ein Rest ist, ausgewählt aus derselben Gruppe wie R, sich von diesem jedoch unterscheidet, und mit dem Fluorid $M_1F_z$ der ersten Sulfonylhalogenidkomponente kontaktiert, wobei man die Mengen der Verbindungen $R'SO_2F$ und $M_1F_z$ so wählt, daß das Molarverhältnis $R'SO_2F : RSO_2Cl$ im wesentlichen gleich 1:1 ist und das Molarverhältnis $RSO_2Cl : M_1F_z$ im wesentlichen höchstens z:1 ist und das gebildete asymmetrische Imid der Formel $M_1(RSO_2NSO_2R')_2$ entspricht.

13. Verfahren nach Anspruch 1, dadurch **gekennzeichnet**, daß man zur Bildung eines asymmetrischen Imids zuerst eine erste Sulfonylhalogenidkomponente, die aus einem Sulfonylfluorid $RSO_2F$ besteht, mit einem Silazanderivat $A((R_2)_3Si)_2N$, das mit einem Fluorid $M_1F_z$ eine Silazankomponente bildet, in solchen Mengen umsetzt, daß das Molarverhältnis $RSO_2F$ : Silazanderivat im wesentlichen 1:1 beträgt, und dann dem erhaltenen Reaktionsmedium einerseits ein Sulfonylchlorid $R'SO_2Cl$, das ebenfalls mit dem Fluorid $M_1F_z$ eine Sulfonylhalogenidkomponente zu bilden vermag, wobei R′ ein Rest ist, ausgewählt aus derselben Gruppe wie R, sich von diesem jedoch unterscheidet, und andererseits das Fluorid $M_1F_z$ zusetzt, wobei das Sulfonylchlorid $R'SO_2Cl$ und das Fluorid $M_1F_z$ in solchen Mengen verwendet werden, daß für den Fall, daß das Silazanderivat die Formel $((R_3)_3Si)_3N$ hat, das Molarverhältnis $R'SO_2Cl$ : Silazanderivat im wesentlichen gleich 1:1 ist und das Molarverhältnis $R'SO_2Cl : M_1F_z$ im wesentlichen höchstens z:2 ist, und für den Fall, daß das Silazanderivat die Formel $((R_2)_3Si)_2)NH$ hat, das Molarverhältnis $R'SO_2Cl$ : Silazanderivat im wesentlichen gleich 1:2 ist und das Molarverhältnis $R'SO_2Cl : M_1F_z$ im wesentlichen höchstens z:2 ist, wobei das erzeugte Imid die Formel $M_1$ $(RSO_2\ N\ SO_2R')_z$ hat. .

14. Verfahren nach einem der Ansprüche 1 bis 13, dadurch gekennzeichnet, daß die Umsetzung der Reagenzien, die zu den Imiden reagieren, in einem aus einem aprotischen polaren Lösungsmittel oder einem Gemisch derartiger Lösungsmittel bestehenden Lösungsmittelmilieu durchgeführt wird.

## Claims

1. Method of synthesising sulphonylimides of the general formula $M((RSO_2)_2N)_y$ in which M is a metal or ammonium radical $N(R_1)_4$, the $R_1$ radicals, which may be identical or different, designating a hydrogen atom or a $C_1$ to $C_8$ hydrocarbyl radical, the R radicals, which may be identical or different, each represent a $C_1$ to $C_{12}$ monovalent organic radical and y is a number equal to the valency of M, in which there is reacted firstly a silazane component containing a silazane compound of the formula $M(((R_2)_3Si)_zN)_y$, in which M and Y have the meanings cited above and $R_2$ is a $C_1$ to $C_4$ alkyl, or a silazane derivative of the formula $A((R_2)_3Si)_2N$ in which A is a hydrogen atom or an $(R_2)_3Si$ radical where $R_2$ has the above meaning, with, secondly, at least one sulphonyl halide component comprising sulphonyl fluoride $RSO_2F$ or a sulphonyl chloride $RSC_2Cl$, R having the meaning indicated above, the said method being characterised in that it consists in bringing the silazane component comprising a silazane compound $M\ [((R_2)_3Si)_2N]_y$ with a sulphonyl halide component comprising a sulphonyl fluoride $RSO_2F$ in a molar ratio of the $RSO_2F$ compound to the silazane compound which is substantially equal to 2y:1 in order to form a symmetrical imide, or in that it employs a silazane component resulting from the combination of a silazane derivative $A\ ((R_2)_3Si)_2N$ and a fluoride $M_1F_z$ and(or at least one sulphonyl halide component resulting from the combination of a sulphonyl chloride $RSO_2Cl$ and a fluoride $M_1F_z$, the said fluoride $M_1F_z$ being a fluoride with low lattice energy for which $M_1$ is selected from the same group as M and may be identical thereto and z is the valency of $M_1$.

2. Method according to Claim 1, characterised in that the imide is produced by reacting a silazane component resulting from the combination of a silazane derivative $A\ ((R_2)_3Si)_2N$ and a fluoride $M_1F_z$ with a single sulphonyl halide component comprising a sulphonyl fluoride $RSO_2F$ or with a first and then a second sulphonyl halide component of which one consists of a sulphonyl fluoride $RSO_2F$ and the other of a sulphonyl fluoride $R'SO_2F$, R′ belonging to the same group as R but being different therefrom, the molar ratio of the silazane derivative to the fluoride $M_1F_2$ being at most substantially equal to z:1 when the symbol A designates a radical $(R_2)_3Si$ or to 2z:1 when the symbol A designates a hydrogen atom.

3. Method according to Claim 1, characterised in that the imide is produced by reacting a silazane component, consisting of a silazane compound $M(((R_2)_3Si)_2N)_y$, on a single sulphonyl halide component resulting from the combination of a sulphonyl chloride $RSO_2Cl$ and a fluoride $M_1F_z$ or on a first then a second sulphonyl halide component, of which one results from the combination of sulphonyl chloride $RSO_2Cl$ and a fluoride $M_1F_z$ whilst the other consists of a sulphonyl fluoride $R'SO_2F$ or results from the combination of a sulphonyl chloride $R'SO_2Cl$ and the fluoride $M_1F_z$, R′ belonging to the same group as R but being different therefrom, the molar ratio of the compound $RSO_2Cl$ or $R'SO_2Cl$ to the fluoride $M_1F_z$ being at most substantially equal to z:1.

4. Method according to Claim 1, characterised in that the imide is produced by reacting a silazane component, resulting from the combination of a silazane derivative $A((R_2)_3Si)_2N$ and a fluoride $M_1F_2$ on a single sulphonyl halide component resulting from the combination of a sulphonyl chloride $RSO_2Cl$ and the fluoride $M_1F_z$ or on a first then a second sulphonyl halide component of which one results from the combination of a sulphonyl chloride $RSO_2Cl$ with the fluoride $M_1F_z$ and the other consists of a sulphonyl fluoride $R'SO_2F$ or results from the combination of a sulphonyl chloride $R'SO_2Cl$ and the fluoride $M_1F_z$, R′ belonging to the same group as R but being different therefrom, the molar amount c of the fluoride $M_1F_z$ to be combined with the molar amounts a of the silazane derivative and b of sulphonyl chloride or chlorides being such that c is at least substantially equal to $\frac{1}{z}$ x(a + b) when the symbol A of the silazane derivative is an $(R_2)_3Si$ radical and $\frac{1}{z}$ x ($\frac{a}{2}$ + b) when the symbol A of the silazane derivative is a hydrogen atom.

5. Method according to Claim 1 or 3, characterised in that the silazane compound $M(((R_2)_3Si)_2N)_y$ is selected from among those for which M represents a metal in the groups I and II in the Periodic System, in particular Li, Na, K, Ag and Mg, or an $NH_4$ or $-N(R_3)_4$ group, $R_3$ designating a $C_1$ to $C_6$ alkyl radical or a phenyl radical.

6. Method according to any one of Claims 1 to 5, characterised in that the sulphonyl fluorides and chlorides are selected from among those in which R and R′ each represent a $C_1$ to $C_{12}$ hydrocarbyl radical and preferably a $C_1$ to $C_{12}$ $R_F$ perfluorohydrocarbyl radical such as a perfluoroalkyl, perfluorocycloalkyl or perfluoroaryl radical.

7. Method according to Claim 6, characterised in that the $R_F$ radical is a $C_1$ to $C_8$ perfluoroalkyl radical or a perfluorophenyl radical.

8. Method according to any one of Claims 1 to 7, characterised in that the $R_2$ radical of the silazane compound is a methyl or ethyl radical and preferably a methyl radical.

9. Method according to any one of Claims 1 to 8, characterised in that the fluoride $M_1F_z$ combined with the silazane derivative and sulphonyl chloride is selected from among the fluorides KF, AgF and $N(R_4)_4F$, the $R_4$ radicals, which are identical or different, designating a hydrogen atom, a $C_1$ to $C_6$ alkyl radical or a phenyl radical.

10. Method according to any one of Claims 2 to 9, characterised in that, in order to form a symmetrical imide, a single sulphonyl halide component is reacted with the silazane component in amounts such that the molar ratio of sulphonyl fluoride $RSO_2F$ or sulphonyl chloride $RSO_2Cl$ of the sulphonyl halide component to the silazane derivative or compound is substantially equal to:

2y:1 when the silazane component contains a compound of the formula $M((R_2)_3Si)_2N)_y$;

2:1 when the silazane component contains a derivative of the formula $A((R_2)_3Si)_2N$ in which A is an $(R_2)_3Si$ radical; and

3:2 when the silazane component contains a derivative of the formula $A((R_2)_3Si)_2N$ in which A is a hydrogen atom.

11. Method according to any one of Claims 2 to 9, characterised in that, in order to form an asymmetrical imide, in a first stage a first sulphonyl halide component is reacted on the silazane component in amounts such that the molar ratio of sulphonyl chloride or fluoride of the sulphonyl halide component to the silazane compound or derivative is substantially equal to:

y:l when the silazane component contains a compound of the formula $M(((R_2)_3Si)_2N)_y$;

1:1 when the silazane component contains a derivative of the formula $A((R_2)_3Si)_2N$; then, in a second stage, the reaction medium produced in the first stage is brought into contact with a second sulphonyl halide component which is different from the first and used in a molar amount which is substantially equal to that of the first sulphonyl halide component when the initial silazane component contains a $M(((R_2)_3Si)_2 N)_y$ compound or an $A((R_2)_3Si)_2N$ derivative in which A in an $(R_2)_3Si$ radical or is substantially equal to half the amount of the first sulphonyl halide component when the initial silazane component contains a derivative $A((R_2)_3Si)_2N$ in which A is a hydrogen atom.

12. Method according to Claim 1, characterised in that, in order to form an asymmetrical imide, sulphonyl chloride $RSO_2Cl$ of a first sulphonyl halide component combining the said sulphonyl chloride and a fluoride $M_1F_z$ is firstly reacted on a silazane component consisting of a $M_1$ $(((R_2)_3Si)_2N)_y$ compound in amounts such that the molar ratio of sulphonyl chloride to silazane compound is substantially equal to z:1, z being the valency of $M_1$, then the resultant reaction medium is brought into contact with a second sulphonyl halide component comprising a sulphonyl fluoride $R'SO_2F$, R′ being a radical selected from the same group as R but different therefrom,

and with the fluoride $M_1F_z$ of the first sulphonyl halide component, using amounts of the compounds $R'SO_2F$ and $M_1F_z$ such that the molar ratio $R'SO_2F:RSO_2Cl$ is substantially equal to 1:1 and the molar ratio $RSO_2Cl$: $M_1F_z$ is substantially at most equal to z:1, the asymmetrical imide formed corresponding to the formula $M_1(RSO_2NSO_2R')_z$.

13. Method according to Claim 1, characterised in that, in order to form an asymmetrical imide, a first sulphonyl halide component comprising a sulphonyl fluoride $RSO_2F$ is firstly reacted with a silazane derivative $A((R_2)_3Si)_2N$ hich forms a silazane component with a fluoride $M_1F_z$ in amounts such that the molar ratio $RSO_2F$: silazane derivative is substantially equal to 1:1, then there is added to the resultant reaction medium firstly a sulphonyl chloride $R'SO_2Cl$, which may also form a sulphonyl halide component with the fluoride $M_1F_z$, R′ being a radical selected from the same group as the radical R but different therefrom, and, secondly, the said fluoride $M_1F_z$ using amounts of sulphonyl chloride $R'SO_2Cl$ and fluoride $M_1F_z$ such that, when the silazane derivative has the formula $((R_3)_3Si)_3N$, the molar ratio $R'SO_2Cl$: silazane derivative is substantially equal to 1:1 and the molar ratio $R'SO_2Cl$: $M_1F_z$ is at most substantially equal to z:2 and that, when the silazane derivative has the formula $((R_2)_3Si)_2)NH$, the molar ratio $R'SO_2Cl$: silazane derivative is substantially equal to 1:2 and the molar ratio $R'SO_2Cl$ : $M_1F_z$ is at most substantially equal to z:2, the imide produced having the formula $M_1$ $(RSO_2NSO_2 R')_z$.

14. Method according to any one of Claims 1 to 13, characterised in that the ingredients which react to form the imides are brought into contact in a solvent medium consisting of a polar aprotic solvent or a mixture of such solvents.